# EUROPEAN PATENT SPECIFICATION

(11) **EP 3 448 445 B1**
(45) Date of publication and mention of the grant of the patent: **06.04.2022**
(21) Application number: 17722960.6
(22) Date of filing: 01.05.2017
(51) Int. Cl.: A61L 27/18, A61L 27/52, A61L 31/06, A61L 31/14

(54) **SPIN TRAP ANTI-ADHESION HYDROGELS**
SPIN-TRAP-ADHÄSIONSSCHUTZHYDROGELE
HYDROGELS ANTI-ADHÉSIFS À PIÈGE DE SPIN

(30) Priority: 30.04.2016 US 201662330100 P
(43) Date of publication of application: 06.03.2019
(73) Proprietor: BVW Holding AG, 6330 Cham (CH)
(72) Inventor: MILBOCKER, Michael, Holliston Massachusetts 01746 (US); BLUECHER, Lukas, 82547 Eurasburg (DE)
(74) Representative: Potter Clarkson
(86) International application number: PCT/US2017/030441
(87) International publication number: WO 2017/190144

(56) References cited:
- US-A- 5 885 566
- US-A1- 2013 196 003
- US-A1- 2015 352 251
- Lipika Basumallick ET AL: "The fate of free radicals in a cellulose based hydrogel: Detection by electron paramagnetic resonance spectroscopy", Journal of Pharmaceutical Sciences, vol. 98, no. 7, 1 July 2009 (2009-07-01), pages 2464-2471, XP55659342, US ISSN: 0022-3549, DOI: 10.1002/jps.21632

## Description

### CROSS-REFERENCE TO RELATED APPLICATIONS

This application claims benefit of U.S. provisional application no. 62/330,100, filed on April 30, 2016.

### FIELD OF THE INVENTION

The present disclosure provides free radical trapping hydrogels and their use thereof in methods for preventing postoperative adhesion formation between organ surfaces.

### BACKGROUND

Abdominal adhesions are bands of fibrous tissue that cause abdominal organs to adhere to one another or to the abdominal wall. Common examples are intestine-tointestine, and intestine-to-pelvic organs, intestine-to-abdominal wall and omentum to any of these sites. Adhesions can develop as aftereffects of peritonitis, or of abdominal trauma. However, such adhesions most commonly result from abdominal surgical procedures during which organs are traumatized by surgical manipulations. Constricting abdominal adhesions can block the flow of contents through the intestines, a condition called intestinal obstruction. In certain instances, a segment of bowel becomes twisted around an adhesive band, thus cutting off the normal blood supply. The affected portion of the intestine becomes nonviable and may perforate. This requires emergency surgery for corrective action. In the U.S., each year about 100,000 operations are carried out to alleviate intestinal obstructions. Once abdominal adhesions have formed, they do not resolve. Their lysis, by operation, only temporarily eliminates them. For example, when surgery is performed for adhesive intestinal obstruction caused by adhesions, adhesions routinely reform and later cause new intestinal obstruction in 11%-21% of such cases. The pathogenesis of adhesion formation is complex and not entirely understood. The first step is believed to involve excess fibrin deposition to form a scaffold. Organization of the fibrin scaffold by cellular elements, including fibroblasts and mesothelial cells, then follows. Adhesion formation is often associated with surgical intervention. The fibrin and various chemical compounds responsible for promoting cellular infiltration into the space between tissue surfaces is present due to damage to tissue surfaces caused by surgical intervention. The polymerization of fibrin into adhesive structures and the subsequent population of these structures by fibroblasts is in part driven by free radicals and oxidative damage.

Various approaches for the prevention of adhesion formation have been actively explored. In general, the treatments fall into three categories: prevention of fibrin deposition in the peritoneal exudate, reduction of local tissue inflammation; and removal of fibrin deposits. Therapeutic attempts to prevent fibrin deposition include peritoneal lavages to dilute or wash away fibrinous exudate, surgical techniques to minimize tissue ischemia and introduction of barriers to limit apposition of healing serosal surfaces.

It has been found that agents promoting polymerization or coagulation of the fibrinous fluid present due to tissue damage promotes adhesion formation [Elkins, T. E. "Can a Pro-Coagulant Substance Prevent Adhesions?" in "Treatment of Post-Surgical Adhesions," diZerega, G. S. et al., eds., Wiley-Liss, N.Y., pp. 103-112 (1990)]. Anti-inflammatory drugs have been evaluated for their effects on postoperative adhesion formation, as they may limit the release of fibrinous exudate after surgical closure. Chronic inflammation at the surgical site due to free radical formation is one factor in promoting continued release of fibrinous exudate. Two general classes of anti-inflammatory drugs have been tested: cortico-steroids and nonsteroidal anti-inflammatory drugs. The results of corticosteroid use in animal studies have generally not been encouraging, probably because clinical use of corticosteroids limited by their other pharmacologic properties. While experimental evaluations of nonsteroidal anti-inflammatory drugs in postoperative adhesion formation show promise [Rodgers, K. E., "Nonsteroidal anti-inflammatory drugs (NSAIDs) in the treatment of Postsurgical adhesion," in "Treatment of Post-Surgical Adhesions," diZerega, G. S. et al., eds., Wiley-Liss, N.Y., pp. 119-129 (1990)].

The third approach involves the removal of fibrin deposits. Although proteolytic enzymes (e.g., pepsin, trypsin and papain) should theoretically augment the local fibrinolytic system and limit adhesion formation, these enzymes are rapidly neutralized by peritoneal exudates rendering them virtually useless for adhesion prophylaxis. While various fibrinolytics (for example, fibrinolysin, streptokinase and urokinase) have been advocated, a potential complication to the clinical use of these enzymes in postoperative therapy is excessive bleeding resulting from their administration. A problem with this approach is that they act on adhesions that have already formed.

Accordingly, it would be preferable to prevent fibrin polymerization before adhesions form. Available products for diminishing the effects of post-surgical adhesions are site specific and are not intended to solve the problem throughout the abdomen. This is a limited benefit because the locations of future adhesions are not entirely predictable. Some of the currently used products include hyaluronic acid and/or carboxy methylcellulose. Some are fabricated as a film, others in a sponge-like configuration, and still others as gels. They must be applied in a selected fashion directly to the surfaces of the specific organs or areas where adhesions might be expected to form or where adhesions would be particularly troublesome, such as over the pelvic organs.

While site-specific blocking of adhesion formation has shown some efficacy, the processes responsible for promoting adhesion are still present and if the product migrates within the body or is removed by absorption, adhesions can subsequently form. US5885566 A discloses a method for modifying a plastic surface of an article, said surface adapted for contact with living tissue of a human or non-human animal, by a gamma-irradiation induced polymerized, chemically grafted coating thereon comprising NVP and HEMA. The aqueous graft polymerization solution can include a free radical scavenger. Modification of medical instruments, devices, implants and the like improves the surfaces thereof so as to improve blood compatibility and reduce tissue adhesion.

The importance of reactive oxygen species in adhesion formation has been demonstrated where no other explanation for adhesion formation is present. For example, pneumoperitoneum used during laparoscopy is a cofactor in adhesion formation. Reactive oxygen species are produced in a hyperoxic environment and during the ischaemia/reperfusion process.

Reactive oxygen activity is deleterious for cells, which protect themselves by an antioxidant system known as ROS scavengers. ROS activity can increase by up-regulation of ROS themselves or by down-regulation of ROS scavengers. It has also been shown that the administration of ROS scavengers decreases adhesion formation in several animal models. ROS activity increases during both laparotomy and laparoscopy. During laparoscopy, the pneumoperitoneum determines ischaemia at the time of insufflation and reperfusion at the time of deflation. During laparotomy, the environment has a 150 mmHg partial pressure of oxygen (pO2), which is much higher than the intracellular pO2 (5-40 mmHg). Researchers attribute the increase in ROS activity to the elevated pO2. Reactive oxygen species are also generated by phagocytic cells at the site of tissue injury or surrounding an implant. Reactive oxygen species serve as major signaling molecules regulating the expression of vascular endothelial growth factor and subsequent wound repair. An experimental model of peritoneal adhesion in rodents designed to study the dynamics of ROS-induced gene expression during de novo adhesion tissue formation has been conducted. Immunohistochemical analysis demonstrated presence of ROS/oxidant and macrophages in the peritoneal tissue. The presence of ROS and ROS-sensitive transcription factor EGR-1 was also evident.

Spin traps are compounds that have the ability to stabilize or 'trap' free radicals, such as reactive oxygen species, thereby reducing the negative cascade effect on other molecules. Although there are over 25 spin traps according to the National Institute of Environmental Health Sciences, the most commonly used spin traps are nitrones, such as alpha-phenyl N-tertiary-butyl nitrone (PBN). This compound is used commercially in skin care products.

Spin trap is considered an 'intelligent' antioxidant because instead of destroying free radicals, it 'traps' them, converts them into harmless and useful oxygen and then transports them back into the respiratory cycle. They are the only antioxidants capable of differentiating between good oxygen molecules and harmful ones. Another commonly used spin trap is 5,5-dimethyl-pyrroline N-oxide (DMPO). More rarely, C-nitroso spin traps are used, such as 3,5-Dibromo-4-nitrosobenzenesulfonic acid (DBNBS). 5-Diisopropoxyphosphoryl-5-methyl-1-pyrroline-N-oxide (DIPPMPO) has been used in trapping superoxide production in mitochondria.

Nitrone spin traps are therapeutic in diverse ways. For example, PBN and derivatives thereof, have been reported for the treatment of a wide variety of inflammatory disease conditions arising from or characterized by free radical-induced oxidative damage. Such disease conditions include, for example, disorders of the central nervous system and the peripheral nervous system, such as stroke, Parkinsonism, traumatic nerve damage and the like, and disorders of the peripheral organs, such as atherosclerosis, cardiac infarction, ulcerative colitis and the like. Nitrones have also been reported to be effective in treating arthritis.

In studies of polymerization of bovine serum albumin by hydroxyl free radicals generated by the Fenton reaction indicated that free ascorbyl palmitate and 5,5-dimethyl-pyrroline N-oxide exert a considerable protective effect against polymerization by scavenging the hydroxyl free radicals. In these studies ascorbyl palmitate was 1 order of magnitude faster in scavenging these radicals than DMPO. Oxidative modification of bovine serum albumin by cobalt gamma irradiation (80 krad) resulted in a strong increase in protein carbonyl content. Ascorbyl palmitate inhibits carbonyl formation very efficiently, indicating that ascorbyl palmitate may inhibit inflammatory processes through multiple pathways.

Accordingly, there is a need for compositions and methods of preventing and treating surgical adhesions. The present disclosure addresses these needs.

### SUMMARY OF THE INVENTION

Provided herein are novel compositions for reducing, inhibiting, preventing or treating the formation of surgical adhesions. These compositions are based, in part, on inventors' discovery that free radical scavengers when covalently bonded to an implantable hydrogel are unexpectedly and surprisingly effective in reducing the incidence of adhesions.

Accordingly, in one aspect, provided herein is a composition according to claim 1 for use in a method of reducing, inhibiting, preventing or treating adhesion formation, the associated method comprising administering to a subject in need thereof a therapeutically effective amount of a free radical scavenger covalently bonded to a hydrogel polymer, a derivative thereof or a prodrug thereof.

The method and associated embodiments described herein can be used to prophylactically prevent post-surgical adhesions. Thus, the method comprises administering to a subject an effective amount of a free radical scavenging hydrogel attached to a soft tissue repair device. For example, the delivery device may be a prosthetic. In particular the delivery device may be a mesh, such as the type used in hernia repair.

In some embodiments, the free radical scavenger is a nitroso compound, or a nitrone compound, or any combination thereof. The hydrogel can be in any suitable form. For example, the hydrogel can be formulated to be bioabsorbable, swellable, possessing low or high Young's modulus, low or high tensile strength, tissue adhesivity and other mechanical characteristics known to the art.

In addition to at least some of the free radical scavenger covalently bonded to the hydrogel polymer, some of the free radical scavenger may be in unbonded or free form. Other compounds may be added to the hydrogel in free or bound form, for example, steroidal and non-steroidal anti-inflammatory compounds. The density of the hydrogel can be varied to realize a controlled-dose or controlled release formulation.

The hydrogel may be incorporated into a physical barrier (e.g., a biodegradable barrier) that is placed in the subject during surgery. The hydrogel may be incorporated into a soft tissue reinforcement device (e.g., a mesh) that is placed in the subject during surgery.

In some embodiments, the free radical scavenging hydrogel can contain a therapeutic agent encapsulated in a liposome or micelle for delayed release of the agent into a subject.

In some embodiments, the free radical scavenging hydrogel can be formulated into a tissue adhesive gel for localized placement in a subject.

In some embodiments, the free radical scavenging hydrogel can be applied to a biodegradable barrier that can be placed in the subject during surgery.

In some embodiments, the subject is a mammal. In some embodiments, the subject is a human.

Generally, the present invention relates to hydrogel-forming, self-solvating, absorbable polymers capable of selective, segmental association into compliant hydrogels, either prior to or upon contacting an aqueous environment.

Disclosed, but not claimed are also methods of using the polymers of the invention in humans for providing a protective barrier to prevent post-surgical adhesion, a carrier of viable cells or living tissue, treatment of defects of the abdomen, and controlled release of biologically active agents for modulating cellular signaling such as wound healing and tissue regeneration or therapeutic treatment of diseases such as cancer and infection.

The present compositions are preferably advantageously used, for example, in the reduction or prevention of adhesion formation subsequent to medical procedures such as surgery and as lubricants and sealants. In addition, compositions according to the present invention may be used as coatings and transient barriers in the body, for materials which control the release of bioactive agents in the body (drug delivery applications).

### BRIEF DESCRIPTION OF THE SEVERAL VIEWS OF THE DRAWINGS

FIG. 1 is a schematic representation of a gel polymer 100 of the present disclosure.
FIG. 2 illustrates a bifurcating polymer sequence 200.
FIG. 3 is a schematic of a mixture of dendritic 302 and comb 304 polymers.

### DETAILED DESCRIPTION OF THE INVENTION

The present invention is based on the discovery that free radical scavenging hydrogels, particularly hydrogels covalently bonded to anti-oxidative or spin trap molecules, can be effectively used to prevent or reduce formation of adhesions between organ surfaces following surgical procedures.

Free radical scavengers are a group of natural and synthetic compounds whose principal effects on target species are to sequester, deactivate or reduce ionic structures in living tissue. Free radical scavengers have also been shown to exert immunomodulatory and anti-inflammatory functions, however the mechanism of these functions are not well understood. The inventive composition of a free radical scavenger covalently bonded to a hydrogel and methods are useful in minimizing or preventing adhesion formation between organ surfaces in body cavities. The most common use of which is peri-operatively. Free radical scavengers coupled to a hydrogel have been found to be especially effective in preventing the formation of adhesion formation in the peritoneum following surgery. In addition, the present invention finds utility in other contexts, e.g., for cardiovascular, orthopedic, thoracic, ophthalmic, CNS and other uses, where prevention of the formation of adhesions is a significant concern. For example, prevention of adhesion formation subsequent to the intraperitoneal administration of a soft tissue repair device is contemplated as within the scope of the present invention. For the purposes of the following discussion, attention is directed primarily to description of compositions and methods useful in inhibiting peritoneal adhesion formation.

An improved anti-adhesion product would be obtained if the inflammation and the free radicals responsible for inflammation are non-site-specifically removed from the interstitial environment. Such a device would preferably possess both a mechanically blocking functionality and a free radical trapping functionality, where the trapping functionality deactivates or sequesters compounds responsible for inflammation, cellular infiltration, and fibrin polymerization.

We have investigated the hypothesis that a free radical scavenger attached to a lubricious and blocking hydrogel meets these clinical needs.

Compounds useful as free radical scavengers are nitroso and nitrone compounds. In general, any spin trap compound is useful in an anti-adhesion device to be implanted in the body. These compounds can mitigate activation of growth factor receptors, extracellular regulated kinases 1 and 2, and p38 kinase because of their ability to prevent reduced glutathione depletion and scavenge hydrogen peroxide. Many of these compounds, in isolation, are less effective due to an effect called the polar paradox. By coupling these compounds covalently to an amphiphilic hydrogel overcomes the issue associated with polarity.

The polar paradox is a theory that illustrates the paradoxical behavior of antioxidants in different media and rationalizes the fact that polar antioxidants are more effective in less polar media, such as bulk oils, while nonpolar antioxidants are more effective in relatively more polar media, such as oil-in-water emulsions or liposomes. By attaching free radical scavengers to amphiphilic polymer chains makes them more effective in both hydrophilic and hydrophobic environments. Hydrogels are three-dimensional polymer networks composed of homopolymers or copolymers that can be comprised of hydrophilic monomers at some locations within the network and lipophilic monomers at other locations.

Another useful characteristic of hydrogels is that they can swell in the body without dissolving. Thus, they provide an absorptive or sponge-like functionality while remaining localized. Their high water content and soft consistency make hydrogels preferable to solid devices, which may cause chronic tissue irritation and promote inflammation.

Many hydrogels are compatible with living systems and hydrogels have found numerous applications in medical and pharmaceutical industries. For example, hydrogels have been investigated widely as drug carriers due to their adjustable swelling capacities, which permit flexible control of drug release rates.

A gel capable of absorbing and then deactivating reactive oxygen species presents a novel approach to preventing post-operative adhesions.

Generally, the present invention relates to composition for preventing adhesions by removing cell signaling and fibrin polymerizing moieties for a period subsequent to a surgical intervention by implanting a hydrogel containing device. More specifically, the invention pertains to the use of nitroso compounds or nitrone compounds covalently bonded in combination or separately to a hydrogel polymer.

In one embodiment, a nitroso compound is covalently bonded to a crosslinked polyurethane/polyurea hydrogel. In another embodiment a nitrone compound is substituted for the nitroso compound. In still another embodiment a free radical scavenging hydrogel of the present invention is incorporated into a polypropylene mesh.

Also provided herein, but not claimed, are methods for inhibiting, reducing, and/or treating adhesion formation or adhesiogenesis. These methods are based on the inventors' discovery that free radical scavengers bonded to a hydrogel are unexpectedly and surprisingly effective in reducing the incidence of adhesions.

In one aspect provided herein is a composition for the treatment and inhibition of adhesions and adhesion formation, the composition comprising the combination of a hydrogel with a free radical scavenger, some of the free radical scavenger in covalently bonded form and optionally some of the free radical scavenger is in free form. Preferably, the free radical scavenger is a spin trap molecule.

Without limitations, the compositions and methods described herein can be used for treating, preventing, or minimizing implant adhesions such as those resulting from natural or synthetic, autologous or heterologous, implants used in breast, abdominal wall, cosmetic, orthopedic, craniofacial, cardiac, or urologic surgeries. Accordingly, the compositions and methods described herein can be used in the treatment of post-operative adhesions, adhesions resulting from trauma, or in patients undergoing surgery that are predisposed to adhesion formation resulting from a fibrotic disease. Further, the treatment can be prophylactic or to prevent a recurrence of an existing adhesion corrected by surgery.

The compositions and methods described herein are particularly useful in the perioperative treatment and inhibition of potential adhesions that might form in the peritoneal or pelvic cavities as a result of a wound, surgical procedure, infection, inflammation, or trauma. The methods described herein have been shown to be especially effective in preventing adhesion formation in the peritoneum following surgery. In addition, the methods described herein find utility in other contexts, e.g., for cardiovascular, orthopedic, thoracic, ophthalmic, CNS and other uses, where prevention of the formation of adhesions is a significant concern and complication following surgery at these sites.

Accordingly, the compositions and methods described herein are useful for the treatment of, inhibition of, suppression of, or reduction of the formation or reformation of adhesions, resulting from wound, surgery, infection, inflammation, trauma, or any combination thereof. Treatment or inhibition of adhesions can include, but is not limited to, a lowering or decrease in the incidence of adhesions, a decrease in the size of adhesions, or a decrease in the rate of adhesiogenesis.

The terms "treat," "treatment," "treating," or "amelioration" refer to therapeutic treatments for adhesion formation, wherein the object is to reverse, alleviate, ameliorate, inhibit, slow down or stop the progression or severity of a symptom or condition associated with adhesion formation. The terms "treat," "treatment," "treating," and "amelioration" include reducing or alleviating at least one adverse effect or symptom of a condition, disease or disorder associated with adhesiogenesis. Treatment is generally "effective" if one or more symptoms or clinical markers are reduced. Alternatively, treatment is "effective" if the progression of a disorder is reduced or halted. That is, "treatment" includes not just the improvement of symptoms or markers of adhesion, but also a cessation or at least slowing of progress or worsening of symptoms of adhesion that would be expected in absence of treatment.

Beneficial or desired clinical results include, but are not limited to, alleviation of one or more symptom(s), diminishment of extent of the disorder, stabilized (i.e., not worsening) state of the disorder, delay or slowing of disorder progression, amelioration or palliation of the disorder state, and remission (whether partial or total), whether detectable or undetectable.

The term "treatment" of a disorder also includes providing reduced severity of the symptoms or side-effects of the disorder (including palliative treatment). By "reduced severity" is meant at least a 10% reduction in the severity or degree of a symptom or measurable disease marker, relative to a control or reference.

As used herein, the terms "prevent," "preventing" and "prevention" refer to the avoidance or delay in manifestation of one or more symptoms or measurable markers of a disease or disorder. A delay in the manifestation of a symptom or marker is a delay relative to the time at which such symptom or marker manifests in a control or untreated subject with a similar likelihood or susceptibility of developing the disease or disorder. The terms "prevent," "preventing" and "prevention" include not only the complete avoidance or prevention of symptoms, but also a reduced severity or degree of any one of those symptoms, relative to those symptoms arising in a control or non-treated individual (e.g. a normally healthy subject) with a similar likelihood or susceptibility of developing the disease or disorder, or relative to symptoms likely to arise based on historical or statistical measures of populations affected by the disease or disorder. The phrase "free radical scavenger" refers to a chemical compound capable of acting as a free radical inactivator or a chemical compound that binds with free radicals to remove them from circulation in a biological environment, e.g., living tissue. A free radical scavenger can be a composition containing a compound that reacts with free radicals in a biological system resulting in the reduction of free radical-induced tissue damage, and protects against the indirect effects of free radicals produced as a result of an tissue insult.

The phrase "spin trap" refers to a molecule or compound with the ability to stabilize, trap or remove free radicals from a cellular environment, and so reduce their cascade effect on other molecules. Spin trap compositions include radical scavengers, and more generally compounds that immobilize free radicals and sequester free radicals in the volume of the spin trap composition. Spin traps are medically characterized by their modulation or regulation of proinflammatory cytokines in living tissue by altering the abundance, concentration or gradient of signalling proteins and glycoproteins employed in cellular communication.

The term "hydrogel" refers to any gel containing water. The term "gel" refers to any composition comprise of one or more of the following: (i) a covalent polymer network, e.g., a network formed by crosslinking polymer chains or by nonlinear polymerization; (ii) a polymer network formed through the physical aggregation of polymer chains, caused by hydrogen bonds, crystallization, helix formation, complexation, etc., that results in regions of local order acting as the network junction points; (iii) a polymer network formed through glassy junction points, e.g., one based on block copolymers; (iv) lamellar structures including mesophases, e.g., soap gels, phospholipids, and clays; (v) particulate disordered structures, e.g., a flocculent precipitate usually consisting of particles with large geometrical anisotropy, such as in V2O5 gels and globular or fibrillar protein gels.

The present disclosure relates to the treatment or prevention of adhesiogenesis resulting from surgery, trauma or injury to tissue. Surgical procedures that can result in tissue injury, especially tissue injury to the peritoneum, include ischemia, electrocautery, and abrasion. These procedures result in a rapid influx of inflammatory cells into the tissue, e.g., peritoneum, which elicits an acute inflammatory response and initiates a cascade of events biologically disposed to facilitate normal wound healing. The inflammatory response can cause a fibrinous exudate to form between adjacent organs. As inflammatory cells infiltrate the fibrinous exudate, the material becomes organized, eventually forming dense bands of fibrous tissue connecting a tissue to an organ, i.e., adhesions. intra-abdominal or peritoneal adhesions occur in more than 94% of patients after abdominal surgery and also arise after pelvic surgeries. Adhesions can result in intestinal blockage, female infertility, chronic pelvic pain and high risk reoperative procedures. Adhesions are also problematic in orthopedic and plastic surgeries, such as in the hand, where impediment of movement is frequently troublesome to the patient.

Accordingly, the term "adhesion," as used herein in the medical sense, refers to a conglutination, or in the chemistry sense a polymerization, resulting in adherence or the uniting of two surfaces or parts within a living body, such as between two organ surfaces. For example, commonly, the union of the opposing surfaces of a wound, or opposing surfaces of peritoneum. Also, adhesions, in the plural, can refer to bands of connective tissue that connect opposing serous surfaces. The term adhesion, as used herein, also includes "fibrinous adhesions," which are adhesions that comprise fine threads of fibrin resulting from an exudate of plasma or lymph, or an extravasation of blood.

"Basal adhesion formation," as used herein in its medical sense, is the basal level of adhesion formation that occurs after wounding (e.g. surgery) or exposure to an atmosphere which contains sufficient oxygen to cause a condition of hypoxia or of hyperoxia.

The compositions and methods described herein are useful in the treatment and prevention of all such adhesions. In some embodiments, an adhesion to be treated or prevented using the methods described herein is one that forms at or occurs between organ surfaces.

As used herein, the term "organ surface" is intended to encompass any internal organ or tissue of a living animal including, but not limited to the uterus, intestine, peritoneum, omentum, stomach, liver, kidneys, heart, and lung.

As used herein, the term "abdominal adhesions" or "peritoneal adhesions" refer to the bands of fibrous tissue that cause abdominal organs to adhere to one another or to the abdominal wall. Postoperative adhesions generally occur as a result of a normal wound healing response within the fifth to seventh day after injury. It is considered that adhesion formation and adhesion-free re-epithelialization are alternative pathways, both of which begin with coagulation, and which initiate a cascade of events resulting in the buildup of fibrin gel matrix. In the case where fibrin deposition is in excess or not removed, the fibrins crosslink to form the fibrin gel matrix, which may then serve as a progenitor or scaffolding on which to form adhesions. The crosslinked scaffold rapidly becomes infiltrated with cellular elements, such as fibroblasts, which produce extracellular matrix materials, such as collagen, which provides the basis for adhesion formation.

In contrast to this, a protective system for fibrinolytic enzyme in peritoneum, such as the plasmin system, can remove the fibrin gel matrix. However, surgery and surgical procedures dramatically attenuate fibrinolytic activity. Therefore it is determined by the environment of the wound site, depending upon the extent of the damage and fibrinolysis in the tissue surface, which pathway of adhesion formation and re-epitheliazation is selected. The present invention is intended to beneficially alter this environment.

Accordingly, in some embodiments, the compositions described herein possess a strong sequestering aspect, in which the balance between fibrin gel formation and fibronolysis is restored. This is one aspect of a method used for the treatment, prevention, inhibition, amelioration, or reduction of post-operative adhesions formed during a surgical procedure, i.e., adhesion formation caused by surgery.

The free radical scavenging hydrogel of the present invention can be combined with grafting or soft tissue reinforcement material. Soft tissue reinforcement materials can comprise biologics such as autologous or autograft material, heterologous materiel, i.e., xenograft material or allograft material, or any combination thereof. Soft tissue reinforcement materials can comprise synthetics such as polypropylene mesh or absorbable constructs such as implants fashioned from polylactic acid.

Examples of grafting material include veins, arteries, heart valves, skin, dermis, epidermis, nerves, tendons, ligaments, bone, bone marrow, blood, white blood cells, red blood cells, gonadocytes, embryos, cells, adipose, fat, muscle, cartilage, fascia, membranes, matrix materials including artificial and/or naturally-occurring components such as, for example, collagen and/or other tissues or components such as, but not limited to, connective tissues, matrix materials including biological or naturally-occurring matrix materials and/or including artificial materials, polymers formed partially or entirely of biological or naturally-occurring materials such as, for example, collagen and/or other tissues or components such as, but not limited to, connective tissues and/or artificial materials, pericardium, plura, periostium, peritoneum, and dura. Implants can include a transplanted organ, such as kidneys, hearts, eyes, and livers, among other things.

The hydrogels of the present invention are polymeric materials with a high tendency for water absorption and/or association, which maintains mechanical integrity through chemical crosslinks or polymeric entanglements which are reversible or degradable in vivo. The hydrophobic blocks may be absorbable polyester chain blocks, polyoxypropylene blocks, urethane segments and botanical extract molecules. Of particular interest are cyclic lactones, for example glycolide, I-lactide, dl-lactide, epsilon-caprolactone, and p dioxanone.

The hydrophilic blocks may be polyoxyethylene blocks, polysaccharides, or derivatives thereof. The length of the hydrophilic block and its weight fractions can be varied to modulate the rate of gel swelling, its modulus, its water content, diffusivity of bioactive drug through it, its adhesiveness to surrounding tissue, and bioabsorbability.

The polymers constructed from these constituents are typically long chains with multiple pendant end groups, commonly referred to as comb- or brush-type copolymers.

The polymer can be biodegradable or non-biodegradable, depending on the intended application. Biodegradable backbones are preferred for most tissue engineering, drug delivery and wound healing device applications, while non-biodegradable backbones are desirable for permanent implant applications.

A portion of the side chains are to be end-capped with free radical scavenging structures functionalized with ligands. In addition to free radical removal from living tissue, cell-signaling can be elicited at the polymer surface or released into the surrounding tissue through degradation of a portion of the polymer.

In one embodiment, the overall comb copolymer should have a molecular weight sufficiently high as to confer good mechanical properties to the polymer in the hydrated state through chain entanglement. That is, its molecular weight should be above the entanglement molecular weight, as defined by one of ordinary skill in the art. The overall molecular weight of the comb copolymer should thus be above about 30,000 Daltons, more preferably above 100,000 Daltons, and more preferably still above 1 million Daltons. In other embodiments, the molecular weight should range from about 30,000 Daltons to about 5 million Daltons, about 100,000 Daltons to about 5 million Daltons, about 1 million Daltons to about 5 million Daltons, about 300,000 Daltons to about 3 million Daltons, or about 1 million to about 3 million Daltons.

The side chains are preferably hydrophilic and degradable, and the polymer backbone contains a multiplicity of hydrophilic, degradable blocks. The density of the hydrophilic side chains along the backbone of the polymers depends on the length of the side chains and the water-solubility characteristics of the final polymer. The total percentage by weight of the hydrophilic side chains is between 10 and 50 percent of the total copolymer composition, preferably around 30 percent by weight. Preferably, the hydrophilic side chains associate with water and form a hydrated layer that repels proteins and hence resists cellular adhesion.

The side chains of a comb polymer can be end-capped with free radical scavenging molecules modified by chemical ligands. Ligands capable of bonding to hydroxyl groups, for example diisocyanates, can be covalently attached to the hydroxyls of free radical scavengers and in turn attached to the hydroxyl groups of the polymer side chains.

A defined fraction of free radical scavenging side chains can be obtained by using appropriate stoichiometric control during the coupling of the ligands to the hydrogel polymers, by protecting the end-groups on those side chains which are not to be end-capped with the free radical scavenging molecule, or by combinations of these approaches. Generally, the ligands are attached to the free radical scavenging molecule first, which then enables the free radical scavenging molecules to link to the polymer side chains without leaving exposed ligands which may promote protein attachment and subsequently adhesions.

The linker group may be varied in chain length depending upon the desired properties. Linkages providing, for example, from 10 to 20 atoms between the backbone and side chain, are typical, although longer linkage chains are possible. Additionally, the linker may be branched to provide for clustering of multiple side chains. These structures are typically referred to as dendritic in structure because they may provide a multiplicity of branching points.

The polymeric backbone section, linkages, side chains and free radical scavenging end groups may be provided in a number of hydrophilic and hydrophobic configurations which will largely determine the stability of the resulting hydrogel. The polymeric backbone itself is comprised of alternating hydrophobic and hydrophilic blocks.

Since the free radical scavenging endgroups are typically hydrophobic, it is generally useful to modify their hydrophobicity by attaching them to hydrophilic side chains.

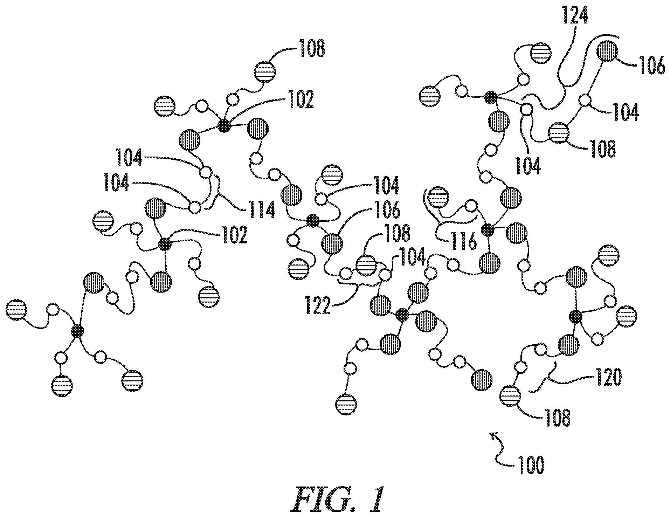

Examples of useful configurations are depicted in FIG. 1 although the invention is not limited to such configurations, and further configurations using the four basic structural units can be provided according to the invention.

FIG. 1 depicts gel polymer 100 of the present invention comprising: a polymeric backbone 102 which defines the overall polymeric morphology, linkage groups 104, side chains 106, and free radical scavenging end groups 108. The backbone 102 is generally comprised of hydrophobic 110 and hydrophilic 112 group segments, some or all of which can be biodegradable. Linkage groups 104 form bridges 114 between the backbones 102, and may be of an entirely different composition than the backbone.

Typically the bridges comprise linkage groups 104 and side chains 106, wherein the backbones 102 are joined to side chains 106 through linkage groups 104. The free radical scavenging group 108 may optionally be located on the ends 116 of the backbone 102, on the ends 118 of pendant side chains 120, sandwiched 124 between two linkage groups 104 and a which in turn links to a side chain 106. Free radical scavenging groups 122 may be located at the junction of two side chains 106 connected by linkage groups 104.

One embodiment comprises polymers wherein the backbone itself is a polyurethane, for example a polyester polyurethane. The side chains, are polyethylene oxide and polypropylene oxide. The side chains are comprised of 75% polyethylene oxide and 25% polypropylene oxide units to which are attached free radical scavenging end groups covalently bonded with a diisocyanate linker.

Dendritic polymers and comb polymer backbones can be provided by the polymerization product of difunctional and higher functional prepolymers. For example linear chains of polyethylene/polypropylene pendant hydroxyl groups can be polymerized with triol endcapped with isocyanate groups. These structures can provide a highly cross-linked polymer which would rapidly degrade to low molecular weight components and readily be cleared by the body.

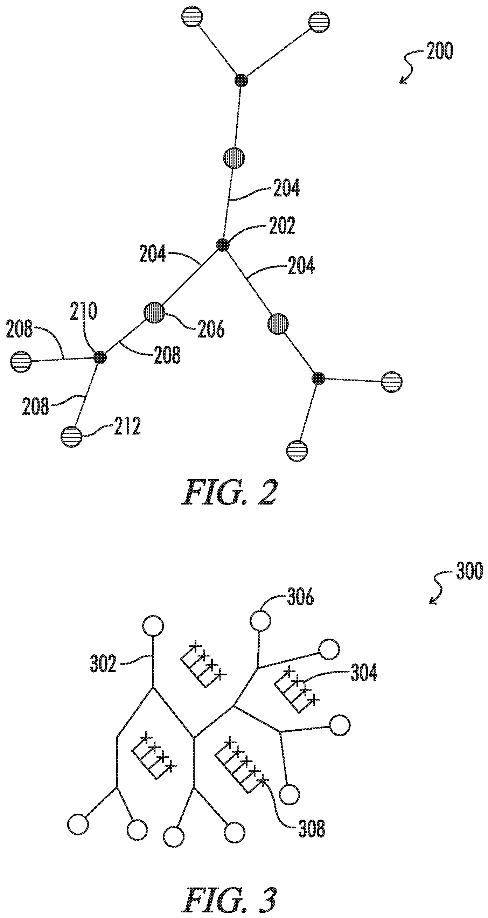

For example, FIG. 2 illustrates a bifurcating sequence 200 wherein a polymer backbone 202 has a 3-armed structure comprising arms 204. The terminus of each arm 204 is linked to via linkage group 206 to another 3-armed structure have arms 208 and backbone 210. At the final terminus of the bifurcating structure are located pendant biofunctional groups 212.

Dendrimers are of particular interest due to their propensity for entanglement and the formation of hydrogels that are relatively stable in the implant environment. Referring to FIG. 3, mixtures 300 of dendritic 302 and comb 304 polymers are possible wherein the dendritic portion serves as a scaffold to the more mobile comb structures. Therefore, the dendritic fraction may be principally endcapped with ascorbyl palmitate end groups 306 and the comb fraction may be endcapped with nitrone end groups 308.

The free radical scavenging hydrogels of the present invention comprise four structural elements: a) a polymeric backbone which defines the overall polymeric morphology, b) linkage groups, c) side chains, and d) free radical scavenging end groups. Referring now to component (a), the polymeric backbone, typically possesses a comb or dendritic morphology comprised of hydrophobic and hydrophilic blocks. The hydrophobic blocks provide volume stability and resistance to degradations; and, the hydrophilic blocks associate water with the polymeric structure and are responsible for imparting the hydrogel aspect to the polymer. In forming gels with water content in excess of 50% by volume, it is desirable that the mass ratio between hydrophobic blocks and hydrophilic blocks be 50:50, and more preferably 30:70.

The volume stability decreases as the proportion of hydrophilic blocks increases. Depending on the constitution of the blocks, if some are hydrolysable, then higher hydrophilic content also correlates with increased degradation rate. In some instances it may be desirable to decouple the degradation properties from the hydrophobicity of the polymeric backbone. In this case, the hydrolyzable units are selected to be the component (b) elements, or the linkage groups.

The hydrolyzable block may be hydrophobic, for example a glycolide, lactide, epsilon-caprolactone, p-dioxanone, or combinations thereof. In general, polyesters are biodegradable. In this case, one might employ an ester of a carboxyl group-containing polysaccharide. For example, a compound formed by bonding at least one of the carboxyl groups of the carboxyl group-containing polysaccharide, preferably at least two of the carboxyl groups of the carboxyl group-containing polysaccharide, with hydroxyl groups of an alcohol to form ester bonds. Among the esterified polysaccharides, those substantially water-soluble are preferable.

The hydrolyzable group may be a polysaccharide, for instance, carboxyl group-containing polysaccharides, such as alginic acid, xanthane gum, gellan gum, derivatives of hyaluronan, and derivatives of polysaccharides which do not have carboxyl groups, such as carboxymethyl cellulose, carboxymethyl dextran and carboxymethyl pullulan; chitin or chitosan derivatives into which carboxyl groups are introduced, such as partially maleylated chitosan, partially succinylated chitosan, carboxymethyl chitosan and carboxymethyl chitin; and the like. Among the above, alginate and hyaluronan are preferable, from the viewpoint of safety and clinically relevant absorption rates.

These polymers are characterized by properties that are a function of the type and ratio of hydrophilic blocks to hydrophobic blocks or structure of mixed hydrophobic/hydrophilic backbone polymers, type and number of tethered free radical scavenging end groups, molecular weight, crosslink density and polymeric morphology.

The polymers of the present invention can be comb- or dendrite-type polymers, with a backbone formed of a hydrophobic, water-insoluble polymer relative to the side chains. Preferably, the side chains comprise short, hydrophilic non-cell binding polymers, having a molecular weight of between 100 and 10,000 Daltons.

The hydrophobic backbone can be biodegradable or non-biodegradable, depending on the desired application. The overall polymer morphology should have a molecular weight sufficiently high to confer stable mechanical properties to the hydrogel polymer through chain entanglement.

Entanglement is a function of molecular weight, morphology and the charge state of various regions on the polymer. The overall hydrophobicity of the polymer also plays a biologic role since association of water with the polymer matrix contributes to mobility of the constituent polymeric components.

Since the free radical scavenging molecules anticipated here are typically hydrophobic, inclusion of them on the terminal sites of these molecules contributes significantly to the overall mechanical stability of the polymer.

The following are exemplary clinically combinations

### Biodegradable Hydrophobic Polymers

Polymers can be both hydrophobic and degradable, but their exclusion of water generally results in a slower degradation rate, when the degradation rate is primarily the result of hydrolysis. Suitable hydrophobic biodegradable polymeric units include hydroxy acids or other biologically degradable polymers that yield degradation products that are non-toxic or present as normal metabolites in the body.

Hydrophobic biodegradable polymeric structures include polyamino acids, polyanhydrides, polyorthoesters, and polyphosphoesters. Polylactones such as polyepsilon-caprolactone, polydelta-valerolactone, polygamma-butyrolactone and polybeta-hydroxybutyrate, for example, are also useful. Preferred polyhydroxy acids are polyglycolic acid, poly DL-lactic acid and poly L-lactic acid, or copolymers of polyglycolic acid and polylactic acid.

In general, these materials degrade in vivo by both non-enzymatic and enzymatic hydrolysis, and by surface or bulk erosion. Any chemical constituents using linkages susceptible to biodegradation is useful in the present invention, and in particular, linkages formed from ester, peptide, anhydride, orthoester and phosphoester bond.

### Non-Biodegradable Hydrophobic Polymers

The preponderance of hydrophobic polymers are non-biodegradable, or at least resist degradation over intervals of months or years. Nevertheless, these structures are useful in the present invention since they can be used in conjunction with hydrolyzable blocks or used to produce polymeric compositions that provide a long-term benefit. These non-biodegradable hydrophobic polymers or polymeric monomers include polyalkylenes such as polyethylene and polypropylene, polychloroprene, polyvinyl ethers, polyvinyl esters such as polyvinyl acetate, polyvinyl halides such as polyvinyl chloride, polysiloxanes, polystyrene, polyurethanes and copolymers thereof,

Hydrophobic moieties with high mechanical strength properties include polyacrylates, such as polymethyl (meth)acrylate, polyethyl (meth)acrylate, polybutyl (meth)acrylate, polyisobutyl (meth)acrylate, polyhexyl (meth)acrylate, polyisodecyl (meth)acrylate, polylauryl (meth)acrylate, polyphenyl (meth)acrylate, polymethyl acrylate, polyisopropyl acrylate, polyisobutyl acrylate, and polyoctadecyl acrylate. To these it is often useful to create polymers comprised of additions of chemical groups, for example, alkyl groups, alkylene groups, hydroxylations, oxidations, and other modifications routinely made by those skilled in the art. Other non-biodegradable polymers include ethylene vinyl acetate, polyacrylates, polychloroprene, and copolymers and mixtures thereof Hydrophilic Side Chains

The side chains are preferably hydrophilic, to modify the typically hydrophobic aspect of free radical scavenging molecules of the present invention. Hydrophilic modification of free radical scavenging constituents affords greater association with biological tissue, such as cells, and in the case of degradation affords ready transport into tissue structures. For example, it may be desirable to construct a polymer with a first function to block tissue adhesions and a second function, subsequent to degradation, of promoting tissue healing and angiogenesis.

The side chains are preferably water-soluble when not attached to the backbone. Suitable polymeric blocks include those prepared from polyoxyethylene, polyoxypropylene, partially or fully hydrolyzed polyvinyl alcohol, polyvinylpyrrolidone, and dextran. The side chains are comprised of 75% of polyoxyethylene and 25% of polyoxypropylene.

Polyoxypropylene is generally not consider hydrophilic, but when copolymerized with polyoxyethylene in a ratio greater than 70:30, polyoxyethylene to polyoxypropylene, the resulting copolymer readily forms stable hydrogels.

The function of the side chains is not necessarily to impart degradability to the overall polymeric structure, thus the hydrophilic side chains may be intrinsically biodegradable or may be poorly biodegradable or effectively non-biodegradable in the body. In the latter two cases, the side chains should be of sufficiently low molecular weight to allow excretion.

When double-bond containing monomers are used to prepare the polymer backbone, apreferred method for incorporating the hydrophilic side chains is to use a hydrophilic macromonomer with a reactive double bond at one end which can be randomly incorporated during free radical or other addition polymerization. An example of such a macromonomer is PEG-methacrylate. The density of the hydrophilic side chains along the polymer backbone can be controlled by adjusting the relative amounts of the PEG-methacrylate or other suitable macromonomeric units used.

It should be noted that in order for the free radical scavenging groups to be attached to a polymeric backbone, one requires appropriate functional groups terminating the side chains, such as -NH₂, --OH, or COOH, to be expressed on the ends of the macromonomers.

### Monomers with Reactive Functional Groups

In many of the embodiments described herein, the monomers used to form the polymer backbone include only two reactive groups, hydroxyl and isocyanate, both of which are reacted in order to form the polymer. For example, lactic acid includes two reactive groups, a hydroxy group and a carboxy group. --OH is the preferred reactive group. Although the ends of a polylactic acid polymer include a hydroxy group and a carboxyl group, there are no reactive groups along the backbone in the final polymer chain that can be used to form a comb copolymer. Therefore, a branching moiety must be incorporated.

Monomers which contain one or more additional reactive groups need to be incorporated into the polymer backbone, preferably in a random fashion, in order to form the comb-type copolymers when monomers that do not include these reactive groups are used to prepare the polymer backbone. Examples of these types of monomers are well known to those of skill in the art.

The requirements for a suitable reactive monomer are that it can be incorporated in the growing polymer chain by participating in the same types of chemical reactions as the growing polymer chain. For example, when lactide is being polymerized using a Lewis acid catalyst, a cyclic dimer of an amino acid can be prepared from lysine, in which the epsilon amine group is protected, for example, with a t-boc protecting group. The lysine is incorporated into the polymer, and the protecting group can be removed. The resulting amine groups are reactive with hydrophilic polymers which include leaving groups such as tosylates, tresylates, mesylates, triflates and other leaving groups well known to those of skill in the art.

Additionally, diamine groups such a biocompatible lysine can be used at polymerizinglinks in isocyanate functionalized polymeric backbones, side chains, and biofunctional end groups.

Alternatively, the reactive monomer can include a leaving group that can be displaced with a nucleophilic group on a hydrophilic polymer. For example, epichlorohydrin can be used during the polymerization step. The monomer is incorporated into the polymer backbone, and the chloride group is present on the backbone for subsequent reaction with nucleophiles. An example of a suitable hydrophilic polymer containing a nucleophilic group is a polyethylene glycol with a terminal amine group. PEG-NH₂ can react with the chloride groups on the polymer backbone to provide a desired density of PEG-ylation on the polymer backbone. Pegylation, in general, is suitable to the spin traps of the present invention, since many of them are poorly incorporated in biological tissue, and can be toxic in the absence of hydrophilic modification.

Using the chemistry described herein, along with the general knowledge of those of skill in the art, one can prepare polymer backbones which include suitable leaving groups or nucleophiles for subsequent coupling reactions with suitably functionalized hydrophilic polymers.

### Ratio of Hydrophilic to Hydrophobic Units

The density of the hydrophilic side chains along the polymer backbone depends in part on the molecular weight of the side chains. The total percent of the hydrophilic units to the hydrophobic units in the present polymers is between 10 and 50 percent by weight, preferably around 30 percent by weight.

One consideration when determining an appropriate ratio of hydrophilic to hydrophobic units is that the overall polymer, when the hydrophilic side chains are not end-capped with free radical scavenging moieties, has some non-cell binding properties and preferably incorporates water around the polymeric construct when implanted in a mammalian body. A relatively high density of 500 Dalton or less hydrophilic side chains can provide the same degree of resistance to cellular adhesion as a lower density of higher molecular weight side chains. Those of skill in the art can adjust the molecular weight and density of the polymers taking these factors into consideration.

### EXAMPLES (Examples 1-27 are not according to the invention)

The materials used in the following examples are available from Sigma-Aldrich, unless otherwise indicated. In some cases equivalent weights are used rather than gram amounts. When equivalent weights are used, the equivalent is defined with respect to a functional group, for example hydroxyl groups, isocyanate groups, amine groups and the like. The relevant functional group should be obvious to one skilled in the art of the synthesis of polymeric gels. When the word "equivalent" is used, it is meant equivalent weight.

### EXAMPLE 1: Poloxamer and polylactic acid based ascorbyl palmitate hydrogel

Pluronic 31R1 (molecular weight 3250) (BASF, Mt. Olive, NJ) was dried under vacuum at 85° C. for 12 hr. in a spherical flask, the final water content obtained was below 300 ppm. One equivalent of Pluronic 31R1 was added to 1/5 equivalent (I)-Lactide and 0.18 grams catalyst (stannous 2-ethyl hexanoate) (0.43%). The reaction was carried out in a sealed flask, under a dry nitrogen saturated atmosphere, for two and half hours at 145 °C. To the above synthesis is added 2 equivalents of toluene diisocyanate and reacted at 60 °C for 8 hours. To this result is added 1/2 equivalent of ascorbyl palmitate, and reacted at 75 °C for 8 hours.

A hydrogel of desired viscosity is formed by adding appropriate amounts of water. For example, for high viscosity gel 1 g water is added, for a low viscosity gel 100 g of water is added.

### EXAMPLE 2: Polyethylene glycol and polylactic acid base ascorbyl palmitate hydrogel

Polyethylene glycol (molecular weight 3000) was dried in vacuo overnight at 85 °C. Thereafter, the PEG was cooled down to room temperature, and the product capped with dry nitrogen. One equivalent of PEG was added to 1/5 equivalent (1)-Lactide and 0.18 grams catalyst (stannous 2-ethyl hexanoate). The mixture of PEG and lactide is placed in an oil bath under flowing nitrogen at 140° C.and mixed for 3 hours.

To the above synthesis is added 2 equivalents of toluene diisocyanate and reacted at 60 ° C for 8 hours. To this result is added 1/2 equivalent of ascorbyl palmitate and reacted at 75 ° C for 8 hours. A hydrogel of desired viscosity is formed by adding appropriate amounts of water. For example, for high viscosity gel 1 g water is added, for a low viscosity gel 100 g of water is added.

### EXAMPLE 3: Poloxamer and polylactic acid based ascorbyl palmitate hydrogel

In a reactor equipped with stir rod, place 2 moles of diisocyanate under nitrogen. Heat the volume to 60 °C and slowly add 1 mole of poloxamer diol. The poloxamer should be added at a rate slow enough such that the volume temperature does not rise above 65 °C. If the poloxamer is a solid at 60 °C, then a solvent can be used. When all the poloxamer has been added to the reaction volume the mixture should be reacted until the isocyanate content corresponds to two available NCO groups per poloxamer molecule. Adding the poloxamer slowly ensures each poloxamer molecule is endcapped with two diisocyanate molecules, because the majority of the reaction is done in an excess of diisocyanate, and chain extension of the poloxamer is less probable. If prevention of chain extension is important a large excess of diisocyanate can be employed, and the excess diisocyanate evaporated at the termination of the reaction.

Once the poloxamer diisocyanate is prepared as described above, 1 mole can be loaded into a reactor under nitrogen, heated to 85 °C and two moles of dilactide (A) or more generally an ester added slowly, and as before preventing an excessive exotherm.

To this product is added 1/2 equivalent of ascorbyl palmitate and reacted at 75 ° C for 8 hours.

A hydrogel of desired viscosity is formed by adding appropriate amounts of water. For example, for high viscosity gel 1 g water is added, for a low viscosity gel 100 g of water is added.

### EXAMPLE 4: Poloxamer and polylactic acid based ascorbyl palmitate hydrogel

While poloxamers of many varied combinations of ethylene oxide (B) and propylene oxide (C) are commercially available, there are practical limits on constructing these chains with monomeric ethylene oxide and propylene oxide. Greater control is afforded by starting with diisocyanates (D) of the monomers, for example DBD or DCD. To these B or C can be arbitrarily added in any combination by forming urethane links between the addition monomer and the diisocyanate end capped chain. Through a step-wise sequence of chain extensions with monomers and subsequent end capping with diisocyanate and combination of B and C can be obtained. One drawback is that the resulting polymer will be more hydrophobic that a chain obtained by direct polymerization of ethylene oxide and propylene oxide. However, this drawback can be compensated in most cases by using less propylene glycol.

Multi-armed polymers can be constructed without crosslinking by introducing a triol (T) and linking the triol to poloxamer chains with diisocyanate. For example, poloxamer chains are introduced into a reactor and endcapped with diisocyanate. The resulting poloxamer diisocyanate is then reacted with a low molecular weight triol such a trimethylolpropane. The result is a poloxamer triisocyanate which then can be reacted with ester (A). Preferably, the ester is polylactic acid.

For every mole of above prepolymer is added 1/10 mole ascorbyl palmitate and reacted at 75 °C for 8 hours.

A hydrogel of desired viscosity is formed by adding appropriate amounts of water. For example, for high viscosity gel 1 g water is added, for a low viscosity gel 100 g of water is added.

### EXAMPLE 5: Absorbable polyurethane based ascorbyl palmitate hydrogel

In a 3-neck flask are placed 400 g of a PLA-Diol (Mn=1000) and 200 g of Terathane 2000 (Invista, Wichita, KS). Toluene is added in excess, and the mixture gently heated to remove toluene to obtain a 20% w/w solution. After cooling to room temperature, 650 g of isophorone diisocyanate was added and mixed under dry nitrogen. To the mixture was then added 5 g of dibutyltin-dilaurate (DBTL) and the mixture was heated to 75 .°C. After 5 hours, 128.7 g of 1,4-butane diol is added and the reaction mixture is diluted with toluene to get concentration of all components of approximately 15% Subsequently, the temperature is raised to 80 °C. After 10 hours the mixture is allowed to cool to room temperature.

For every mole of above prepolymer is added 1/10 mole ascorbyl palmitate and reacted at 75 °C for 8 hours.

A hydrogel of desired viscosity is formed by adding appropriate amounts of water. For example, for high viscosity gel 1 g water is added, for a low viscosity gel 100 g of water is added. After hydrogel formation, the toluene is driven in an excess of water to obtain a solvent free hydrogel.

### EXAMPLE 6: Absorbable polyurethane based ascorbyl palmitate hydrogel

In a 3-neck flask are placed 400 g of a PLA-Diol (Mn=1000) and 400 g of polyethylene glycol (Mn=2000). Toluene is added in excess, and the mixture gently heated to remove toluene to obtain a 20% w/w solution. After cooling to room temperature, 650 g of isophorone diisocyanate was added and mixed under dry nitrogen. To the mixture was then added 5 g of dibutyltin-dilaurate (DBTL) and the mixture was heated to 75 ° C. After 5 hours, 128.5 g of 1,4-butane diol is added and the reaction mixture is diluted with toluene to get concentration of all components of approximately 15%.

Subsequently, the temperature is raised to 80 °C. After 10 hours the mixture is allowed to cool to room temperature to yield the prepolymer.

For every mole of the above prepolymer is added 1/10 mole ascorbyl palmitate and reacted at 75 ° C for 8 hours.

A hydrogel of desired viscosity is formed by adding appropriate amounts of water. For example, for high viscosity gel 1 g water is added, for a low viscosity gel 100 g of water is added. After hydrogel formation, the toluene is driven in an excess of water to obtain a solvent free hydrogel.

### EXAMPLE 7: Absorbable polyurethane based ascorbyl palmitate hydrogel

In a 3-neck flask are placed 400 g of a PLA-Diol (Mn=1000) and 400 g of polyethylene glycol (Mn=2000). Toluene is added in excess, and the mixture gently heated to remove toluene to obtain a 20% w/w solution. After cooling to room temperature, 550 g of isophorone diisocyanate was added and mixed under dry nitrogen. To the mixture was then added 5 g of dibutyltin-dilaurate (DBTL) and the mixture was heated to 75 ° C. After 5 hours, 108.3 g of 1,4-butane diol is added and the reaction mixture is diluted with toluene to get concentration of all components of approximately 15%.

Subsequently, the temperature is raised to 80° C. After 10 hours the mixture is allowed to cool to room temperature to yield a prepolmer.

For every mole of above prepolymer is added 1/10 mole ascorbyl palmitate and reacted at 75 °C for 8 hours.

A hydrogel of desired viscosity is formed by adding appropriate amounts of water. For example, for high viscosity gel 1 g water is added, for a low viscosity gel 100 g of water is added. After hydrogel formation, the toluene is driven in an excess of water to obtain a solvent free hydrogel.

### EXAMPLE 8: Absorbable polyurethane based ascorbyl palmitate hydrogel

In a 3-neck flask are placed 400 g of a PLA-Diol (Mn=1000) and 500 g of polyethylene glycol (Mn=2000). Toluene is added in excess, and the mixture gently heated to remove toluene to obtain a 20% w/w solution. After cooling to room temperature, 512 g of isophorone diisocyanate was added and mixed under dry nitrogen. To the mixture was then added 7 g of dibutyltin-dilaurate (DBTL) and the mixture was heated to 75 °C. After 5 hours, 109.5 g of 1,4-butane diol is added and the reaction mixture is diluted with toluene to get concentration of all components of approximately 15%.

Subsequently, the temperature is raised to 80 ° C. After 10 hours the mixture is allowed to cool to room temperature to yield the prepolymer.

For every mole of above prepolymer is added 1/10 mole ascorbyl palmitate and reacted at 75 °C for 8 hours.

A hydrogel of desired viscosity is formed by adding appropriate amounts of water. For example, for high viscosity gel 1 g water is added, for a low viscosity gel 100 g of water is added. After hydrogel formation, the toluene is driven in an excess of water to obtain a solvent free hydrogel.

### EXAMPLE 9: Absorbable polyurethane based ascorbyl palmitate hydrogel

In a 3-neck flask are placed 765 g of a PLA-Diol (Mn=1000) and 765 g of polyethylene glycol (Mn=2000). Toluene is added in excess, and the mixture gently heated to remove toluene to obtain a 20% w/w solution. After cooling to room temperature, 955 g of isophorone diisocyanate was added and mixed under dry nitrogen. To the mixture was then added 8 g of dibutyltin-dilaurate (DBTL) and the mixture was heated to 75 °C. After 5 hours, 245 g of 1,4-butane diol is added and the reaction mixture is diluted with toluene to get concentration of all components of approximately 15%.

Subsequently, the temperature is raised to 80 ° C. After 10 hours the mixture is allowed to cool to room temperature to yield the prepolymer.

For every mole of above prepolymer is added 1/10 mole ascorbyl palmitate and reacted at 75 °C for 8 hours.

A hydrogel of desired viscosity is formed by adding appropriate amounts of water. For example, for high viscosity gel 1 g water is added, for a low viscosity gel 100 g of water is added. After hydrogel formation, the toluene is driven in an excess of water to obtain a solvent free hydrogel.

### EXAMPLE 10: Absorbable polyurethane based ascorbyl palmitate hydrogel

In a 3-neck flask was placed 2100 g of Terathane 2000. Toluene is added in excess, and the mixture gently heated to remove toluene to obtain a 20% w/w solution. After cooling to room temperature, 814 g of isophorone diisocyanate was added and mixed under dry nitrogen. To the mixture was then added 4 g of dibutyltin-dilaurate (DBTL) and the mixture was heated to 75 °C. After 5 hours, 193 g of 1,4-butane diol is added and the reaction mixture is diluted with toluene to get concentration of all components of approximately 15%. Subsequently, the temperature is raised to 80 °C. After 10 hours the mixture is allowed to cool to room temperature.

For every mole of above prepolymer is added 1/10 mole ascorbyl palmitate and reacted at 75 ° C for 8 hours.

A hydrogel of desired viscosity is formed by adding appropriate amounts of water. For example, for high viscosity gel 1 g water is added, for a low viscosity gel 100 g of water is added. After hydrogel formation, the toluene is driven in an excess of water to obtain a solvent free hydrogel.

### EXAMPLE 11: Absorbable polyurethane based ascorbyl palmitate hydrogel

In a 3-neck flask was placed 400 g of a PLA-Diol (Mn=2000), 200 g of Terathane 2000 and 200 g of polyethylene glycol (Mn=2000). Toluene is added in excess, and the mixture gently heated to remove toluene to obtain a 20% w/w solution. After cooling to room temperature, 505 g of isophorone diisocyanate was added and mixed under dry nitrogen. To the mixture was then added 7 g of dibutyltin-dilaurate (DBTL) and the mixture was heated to 75 °C. After 5 hours, 128.5 g of 1,4-butane diol is added and the reaction mixture is diluted with toluene to get concentration of all components of approximately 15%. Subsequently, the temperature is raised to 80 °C. After 10 hours the mixture is allowed to cool to room temperature to yield the prepolymer.

For every mole of above prepolymer is added 1/10 mole ascorbyl palmitate and reacted at 75 °C for 8 hours.

A hydrogel of desired viscosity is formed by adding appropriate amounts of water. For example, for high viscosity gel 1 g water is added, for a low viscosity gel 100 g of water is added. After hydrogel formation, the toluene is driven in an excess of water to obtain a solvent free hydrogel.

### EXAMPLE 12: Absorbable polyurethane based ascorbyl palmitate hydrogel

In a 3-neck flask was placed 200 g of a PLA-Diol (Mn=2000), 200 g of polycaprolactone (Mn=2000) and 400 g of polyethylene glycol (Mn=2000). Toluene is added in excess, and the mixture gently heated to remove toluene to obtain a 20% w/w solution. After cooling to room temperature, 505 g of isophorone diisocyanate was added and mixed under dry nitrogen. To the mixture was then added 7 g of dibutyltin-dilaurate (DBTL) and the mixture was heated to 75 degrees C. After 5 hours, 128.5 g of 1,4-butane diol is added and the reaction mixture is diluted with toluene to get concentration of all components of approximately 15%. Subsequently, the temperature is raised to 80 ° C. After 10 hours the mixture is allowed to cool to room temperature to yield the prepolymer.

For every mole of above prepolymer is added 1/10 mole ascorbyl palmitate and reacted at 75 °C for 8 hours.

A hydrogel of desired viscosity is formed by adding appropriate amounts of water. For example, for high viscosity gel 1 g water is added, for a low viscosity gel 100 g of water is added. After hydrogel formation, the toluene is driven in an excess of water to obtain a solvent free hydrogel.

### EXAMPLE 13: Absorbable Polyurethane based ascorbyl palmitate hydrogel

Nine hundred grams of Oxymer M112 (carbonate diol, Mn=1500) (Perstorp Specialty Chemicals AB, Perstorp, Sweden) are put into a 3-neck-flask. Toluene is added and partly removed by distillation to get a 20% solution. After cooling to room temperature 96.2 g of hexamethylene diisocyanate are added under nitrogen. 6 g of DBTL are added and the mixture is heated to 75°C. After 5 hours the temperature is raised to 80°C. After 10 hours the mixture is allowed to cool to room temperature to yield the prepolymer.

For every mole of above prepolymer is added 1/10 mole ascorbyl palmitate and reacted at 75 degrees C for 8 hours.

A hydrogel of desired viscosity is formed by adding appropriate amounts of water. For example, for high viscosity gel 1 g water is added, for a low viscosity gel 100 g of water is added. After hydrogel formation, the toluene is driven in an excess of water to obtain a solvent free hydrogel.

### EXAMPLE 15: Absorbable polyurethane based ascorbyl palmitate hydrogel

Six hundred grams of Desmophen 2100 (carbonate diol, Mn=1000) (Bayer, Morristown, NJ) are put into a 3-neck-flask. Toluene is added and partly removed by distillation to get a 20% solution. After cooling to room temperature 181.3 g of isophorone diisocyanate is added under nitrogen. 6 g of DBTL are added and the mixture is heated to 75°C. After 5 hours the temperature is raised to 80°C. After 10 hours the mixture is allowed to cool to room temperature to yield a prepolymer.

For every mole of above prepolymer is added 1/10 mole ascorbyl palmitate and reacted at 75 °C for 8 hours.

A hydrogel of desired viscosity is formed by adding appropriate amounts of water. For example, for high viscosity gel 1 g water is added, for a low viscosity gel 100 g of water is added. After hydrogel formation, the toluene is driven in an excess of water to obtain a solvent free hydrogel.

### EXAMPLE 15: Absorbable polyurethane based ascorbyl palmitate gel

Seven hundred grams of Terathane 2000 (Invista, Wichita, KS) are put into a 3-neck-flask. Toluene is added and then a part of the toluene is removed by distillation to get a 20% solution. After cooling to room temperature, 288 g of isophorone diisocyanate are added under nitrogen. 6 g of DBTL are added and the mixture is heated to 75°C. After 5 hours 128.7 g of 1,4-butane diol are added and the reaction mixture is diluted with toluene to get concentration of all components of 15%. The temperature is raised to 80°C. After 10 hours the mixture is allowed to cool to room temperature to yield a prepolymer.

For every mole of above prepolymer is added 1/10 mole ascorbyl palmitate and reacted at 75 °C for 8 hours.

A hydrogel of desired viscosity is formed by adding appropriate amounts of water. For example, for high viscosity gel 1 g water is added, for a low viscosity gel 100 g of water is added. After hydrogel formation, the toluene is driven in an excess of water to obtain a solvent free hydrogel.

### EXAMPLE 16: Absorbable polyurethane based ascorbyl palmitate hydrogel

Four thousand two hundred grams of Terathane 2000 are put into a 3-neck-flask. Toluene is added and then a part of the toluene is removed by distillation to get a 20% solution. After cooling to room temperature 1514 g of isophorone diisocyanate are added under nitrogen. 7 g of DBTL are added and the mixture is heated to 75°C. After 4 hours 617 g of 1,4-bis(N-methyl)amino cyclohexane are added and the reaction mixture is diluted with toluene to get concentration of all components of 10% The temperature is raised to 80°C. After 8 hours the mixture is allowed to cool to room temperature.

For every mole of above prepolymer is added 1/10 mole ascorbyl palmitate and reacted at 75 °C for 8 hours.

A hydrogel of desired viscosity is formed by adding appropriate amounts of water. For example, for high viscosity gel 1 g water is added, for a low viscosity gel 100 g of water is added. After hydrogel formation, the toluene is driven in an excess of water to obtain a solvent free hydrogel.

### EXAMPLE 17: Absorbable polyurethane based ascorbyl palmitate hydrogel

Four hundred grams of Terathane 2000 and 400 g of polycaprolactone diol (Mn=2000) are put into a 3-neck-flask. Toluene is added and then a part of the toluene is removed by distillation to get a 20% solution. After cooling to room temperature, 505 g of isophorone diisocyanate are added under nitrogen. 7 g of DBTL are added and the mixture is heated to 75°C. After 5 hours 128 g of 1,4-butane diol are added and the reaction mixture is diluted with toluene to get concentration of all components of 15%. The temperature is raised to 80°C. After 10 hours the mixture is allowed to cool to room temperature to yield the prepolymer.

For every mole of above prepolymer is added 1/10 mole ascorbyl palmitate and reacted at 75 ° C for 8 hours.

A hydrogel of desired viscosity is formed by adding appropriate amounts of water. For example, for high viscosity gel 1 g water is added, for a low viscosity gel 100 g of water is added. After hydrogel formation, the toluene is driven in an excess of water to obtain a solvent free hydrogel.

### EXAMPLE 18: Absorbable polyurethane based ascorbyl palmitate hydrogel

476 g of Terathane 2000 and 600 g of polycaprolactone diol (Mn=2000) are put into a 3-neck-flask. Toluene is added and then a part of the toluene is removed by distillation to get a 20% solution. After cooling to room temperature, 404 g of isophorone diisocyanate are added under nitrogen. 4 g of DBTL are added and the mixture is heated to 75°C. After 5 hours 93 g of 1,4-butane diol are added and the reaction mixture is diluted with toluene to get concentration of all components of a 15%. The temperature is raised to 80°C. After 10 hours the mixture is allowed to cool to room temperature to yield the prepolymer.

For every mole of above prepolymer is added 1/10 mole ascorbyl palmitate and reacted at 75 ° C for 8 hours.

A hydrogel of desired viscosity is formed by adding appropriate amounts of water. For example, for high viscosity gel 1 g water is added, for a low viscosity gel 100 g of water is added. After hydrogel formation, the toluene is driven in an excess of water to obtain a solvent free hydrogel.

### EXAMPLE 19: Substituting a carbamate link for a urea link

Any of the examples of polymers described thus far can be functionalized by addition of a terminal amine group suitable for attachment of a free radical scavenging substance. One approach is to amine terminate an ether diol and then polymerize this reaction product with an ester.

An amine terminated PEG can be synthesized by dissolving the PEG into dry THF at -79 ° C utilizing dry ice and methanol as a cooling bath. The amount of amine termination is calculated, and the equivalent amount of 0.25 M solution of potassium-bis-(trimethylsilyl) amide in toluene is then added slowly.

The reaction mixture is then stirred at 20 °C for 48 hours. The reaction product is then diluted 10:1 with ether forming a precipitate which can be subsequently separated from solution by filtration. The precipitate is then dissolved in THF and 0.1N hydrochloric acid was added to split the silylamide. The solution is then stirred for 1 hour at room temperature, and then the polymer is precipitated in ether.

The resulting NH2-PEG can be polymerized with cyclic DL-dilactide. In the desired ratio, the two ingredients are dissolved separately in dry toluene. The polymerization is accomplished by combining the two solutions under dry nitrogen and heating to boiling.

When boil is reached, tin catalyst (tin-2-ethylhexanoate) is then added and reacted for 8 hours. The resulting polymer solution is cooled and mixed with dichloromethane to remove water by evaporation. The dicholoromethane of the dry solution is exchanged with acetone and the resulting solution dripped into distilled water at 0 degree C and the resulting precipitate collected.

### EXAMPLE 20: Polyethylene oxide/polypropylene oxide backbone for a ascorbyl palmitate hydrogel.

Any of the alcohols used in the above examples may be substituted with polyethylene oxide/polypropylene oxide as synthesized below. Water soluble triblock copolymers of polyethylene oxide (PEO) and polypropylene oxide (PPO) are commercially available non-ionic macromolecular surface active agents.

Variation of the copolymer composition (PPO/PEO ratio) and molecular weight (PEO and PPO block lengths) during synthesis leads to the production of molecules with optimum properties. Unfortunately, commercially available forms employ block structures that are typically larger than are most desired for the present invention.

Since PEO is more reactive than PPO fine scale block structures cannot be formed by merely placing the ratio amounts of PEO and PPO together in a reactor. Alternating segments of PEO and PPO can be synthesized by the sequential addition of first propylene oxide (PO) and then ethylene oxide (EO). These oxyalkylation steps are carried out in the presence of an alkaline catalyst, for example, sodium or potassium hydroxide. The catalyst is then neutralized and removed from the final product. By alternating additions of EO and PO one can make copolymers of particular PPO/PEO composition while varying the molecular weight of the PPO blocks. Thus a complete grid of copolymers are realizable, the grid comprised of constant PPO/PEO composition on the vertical axis and constant PPO block molecular weight on the horizontal axis.

### EXAMPLE 21: Hyaluronan Isocyanate functional groups for synthesizing ascorbyl palmitate hydrogel.

Any of the isocyanate components in the above may be substituted with the below described hyaluronan Isocyanate. Hyaluronan is comprised of repeating segments of C14H21NO11, each containing 5 hydroxyl groups (OH). To form a diisocyanate of hyaluronan one reacts a quantity of diisocyanate containing 2 moles of NCO greater than the number of moles of OH. Thus, a hyaluronan containing 1 unit of C14H21NO11 per molecule, then 1 mole of hyaluronan molecules if to be reacted with 7 moles of diisocyanate. The reaction is performed in an organic solvent, where the hyaluronan is altered by ammonia to make it soluble in an organic solvent, for example tetrahydrofuran. A small amount of tin catalyst is added to promote urethane link formation between the hydroxyls of the hyaluronan and the isocyanate groups of the diisocyanate. To discourage chain extension, the hyaluronan is first dissolved in organic solvent and set aside. The reactor is charged with catalyst and diisocyanate and heated to 80 degrees C. The hyaluronan solution is slowly added to the reactor and the exotherm monitored. Complete reaction is indicated when the exotherm subsides. Alternatively, one can measure the %NCO at each step to verify all the hydroxyl groups on the hyaluronan are endcapped with isocyanate.

When all the hyaluronan is added to the reactor the reaction is run until the desired %NCO is reached. %NCO is measured by conventionally by dibutylamine titration. The

reaction is complete when 2 moles of NCO are measured for every mole of product molecule. Ideally there is only 1 C14H21NO11 unit per product molecule. However, in other applications a spectrum of product molecules containing a range of C14H21NO11 unit per product molecule is desired. The desired polydispersity can be obtained by adjusting the amount of NCO used, and verifying with GPC and %NCO measurements. In any one reaction, the dispersity of molecular weights of product molecules will be Gaussian around a desired mean. Multi-modal distributions can be obtained by mixing the reaction product of multiple reactions. Hyaluronan isocyanates of higher isocyanate functionality can be synthesized by adjusting the ratio of OH groups to isocyanate groups in the reaction mix.

### EXAMPLE 22: Hyaluronan polyurethane based ascorbyl palmitate hydrogel

A polyalkylene copolymer of PPO and PEO is synthesized according to EXAMPLE 20 wherein the PEO blocks contain 3 propylene oxide units, the PPO blocks contain 1 ethylene oxide unit, and these PEO and PPO blocks alternate, wherein the first block is a PEO and the last block is a PPO. The number of functional OH groups per molecule is approximately 2.

A hyaluronan diisocyanate is synthesized according to EXAMPLE 21 wherein the molecular weight of the hyaluronan diisocyanate is approximately 3 times the molecular weight of the polyalkylene copolymer. If the polyalkylene component or the hyaluronan diisocyanate components are not in liquid form at a reaction temperature of approximately 80 degrees C, then these components are dissolved in an organic solvent devoid of OH groups.

The reactor is charged with 1 mole of hyaluronan diisocyanate and heated to 80 °C. The polalkylene copolymer is added slowly, waiting for the exotherm to subside after each addition. If a prepolymer is desired, a reaction product that will polymerize on a mesh, then the component amounts are chosen to result in 2 moles of NCO per product molecule.

Chains of arbitrary length of hyaluronan and polyalkylene can be synthesized by choosing the amount of isocyanate such that 2 moles of NCO remain per desired molecular weight of product molecule. In some cases, a prepolymer with 3 or higher isocyanate functionality per product molecule is desired, so that when polymerized on a medical device the coating is resistant to solvents or heat. Not every molecule must have higher functionality to obtain a polymerization product that is crosslinked.

If a linear polymer is desired, wherein the reaction product can be dissolved in solvent and solution cast, or melted and extruded, then some of the hyaluronan diisocyanate must be endcapped with a mono-functional alcohol such as ethanol. The molecular weight of the reaction product is selected by the ratio of diisocyanate to mono-isocyanate hyaluronan in the reaction mix.

Alternatively, the chain extension can be terminated in reaction by adding ethanol to the reaction mix when the desired molecular weight is obtained. In this instance an excess of ethanol can be used, which is driven off by evaporation when all the NCO groups are consumed.

Dibutylamine titration can be used to determine when a reaction is done. In particular, in the polymer case, the reaction is complete when all NCO groups are consumed. In the prepolymer case, the reaction is complete when the NCO number per product molecule reaches a desired value. In the case of crosslinking prepolymers the NCO number is greater than 2 per product molecule. In the case of non-crosslinking prepolymers the NCO number equals 2 per product molecule. The product molecules and polymerized forms are characterized by possessing in number ratio approximately 3 segments of hyaluronan per segment of polyalkylene. The polyalkylene segment comprises in number ratio approximately 3 segments of ethylene oxide per segment of propylene oxide.

The hyaluronan segment is more hydrophilic than the polyalkylene segment. The ethylene oxide segment is more hydrophilic than the propylene oxide segment. The urethane links between hyaluronan units having a molecular weight ratio of urethane to hyaluronan approximately the same as the molecular weight ratio of urethane to polyalkylene segments. The urethane links is more hydrophobic than the hyaluronan units or polyalkylene segments. The density of which can be tailored to form hard segment association between urethane links within the bulk volume of the polymer. For every mole of the above prepolymer is added 1/10 mole ascorbyl palmitate and reacted at 75 °C for 8 hours.

A hydrogel of desired viscosity is formed by adding appropriate amounts of water. For example, for high viscosity gel 1 g water is added, for a low viscosity gel 100 g of water is added. After hydrogel formation, the toluene is driven in an excess of water to obtain a solvent free hydrogel.

### Example 23: Polyester diisocyanate functional groups for synthesizing ascorbyl palmitate hydrogel

In this example a castor-derived hydroxyl-terminated ricinoleate derivative is used as the diol. One equivalent of polycin D-265 (212 g) is combined with 2 equivalent of toluene diisocyanate (174 g) at room temperature (22 degrees C). The mixture is stirred at 100 revolutions per minute and the temperature monitored. The mixture will begin to heat up by exothermic reaction and no heat is to be applied to the reactor until the temperature in the reactor ceases to rise. Then the mixture temperature should be increased in 5 degrees C increments per ½ hour until the mixture reaches 60 °C. The reaction should be continued until the %NCO = 10.9 % . The target % NCO is reached when every hydroxyl group in the mixture is reacted with an NCO group. Ideally, the result is a single diol endcapped with two diisocyanates. This outcome can be enhanced by slow addition of the diol to the diisocyanate. The addition should be in 10 g increments, added when the exotherm from the previous addition has ceased. However, chain extended variations of the above ideal outcome are useful, their primary disadvantage being that the product is slightly higher in viscosity. The ideal %NCO is calculated by dividing the weight of the functional isocyanate groups (2 X 42 Dalton) per product molecule by the total weight of the product molecule (424 Dalton + 2X174 Dalton) yielding approximately 10.9%.

Alternatively, a lower molecular weight diol may be used, such as polycin D-290 where 1 equivalent of polycin D-290 is 193 g and the target %NCO is 84/(386 + 348) = 11.4%.

Alternatively, a higher molecular weight diol may be used, such as polycin D-140 where 1 equivalent of polycin D-140 is 400 g and the target %NCO is 84/(800 + 348) = 7.3%.

All polycin diols are available from Performance Materials (Greensboro, NC) and toluene diisocyanate is available from Sigma-Aldrich (Milwaukee, WI).

### Example 24: Polyether diisocyanate functional groups for synthesizing ascorbyl palmitate hydrogel

In this example a polyether hydroxyl-terminated copolymer of 75% ethylene oxide and 35% propylene oxide is used as the diol. One equivalent of UCON 75-H-450 (490 g) is combined with 2 equivalent of toluene diisocyanate (174 g) at room temperature (22 degrees C). The mixture is stirred at 100 revolutions per minute and the temperature monitored. The mixture will begin to heat up by exothermic reaction and no heat is to be applied to the reactor until the temperature in the reactor ceases to rise. Then the mixture temperature should be increased in 5 degrees C increments per ½ hour until the mixture reaches 60 degrees C. The reaction should be continued until the %NCO = 10.9% . The target % NCO is reached when every hydroxyl group in the mixture is reacted with an NCO group. Ideally, the result is a single diol endcapped with two diisocyanates.

This outcome can be enhanced by slow addition of the diol to the diisocyanate. The addition should be in 10 g increments, added when the exotherm from the previous addition has ceased. However, chain extended variations of the above ideal outcome are useful, their primary disadvantage being that the product is slightly higher in viscosity. The ideal %NCO is calculated by dividing the weight of the functional isocyanate groups (2 X 42 Dalton) per product molecule by the total weight of the product molecule (980 Dalton + 2X174 Dalton) yielding approximately 6.3 %. Polyether copolymers of ethylene oxide and propylene oxide diols are available from Dow Chemical (Midland, MI).

### Example 25: Polyester triisocyanate functional groups for synthesizing ascorbyl palmitate hydrogel

In this example a castor-derived hydroxyl-terminated ricinoleate derivative is used as the triol. One equivalent of polycin T-400 (141 g) is combined with 2 equivalent of toluene diisocyanate (174 g) at room temperature (22 degrees C). The mixture is stirred at 100 revolutions per minute and the temperature monitored. The mixture will begin to heat up by exothermic reaction and no heat is to be applied to the reactor until the temperature in the reactor ceases to rise. Then the mixture temperature should be increased in 5 degrees C increments per ½ hour until the mixture reaches 60 degrees C. The reaction should be continued until the %NCO = 13.3 % . The target % NCO is reached when every hydroxyl group in the mixture is reacted with an NCO group.

Ideally, the result is a single diol endcapped with two diisocyanates. This outcome can be enhanced by slow addition of the diol to the diisocyanate. The addition should be in 10 g increments, added when the exotherm from the previous addition has ceased. However, chain extended variations of the above ideal outcome are useful, their primary disadvantage being that the product is slightly higher in viscosity. The ideal %NCO is calculated by dividing the weight of the functional isocyanate groups (2 X 42 Dalton) per product molecule by the total weight of the product molecule (282 Dalton + 2X174 Dalton) yielding approximately 13.3%.

The above reaction will yield a viscous product. A less viscous product can be obtained by adding propylene carbonate to the initial mixture. Additions up to 100% by weight of propylene carbonate are useful. Adjustment to the target NCO of the mixture must be performed using standard methods, or the propylene carbonate may be added after reaching the target %NCO. Propylene carbonate is available from Sigma-Aldrich (Milwaukee, WI).

### Example 26: Polyether triisocyanate functional groups for synthesizing ascorbyl palmitate hydrogel

In this example a polyether hydroxyl-terminated copolymer of 75% ethylene oxide and 35% propylene oxide is used as the triol. One equivalent of Multranol 9199 (3066 g) is combined with 3 equivalent of toluene diisocyanate (261 g) at room temperature (22 degrees C). The mixture is stirred at 100 revolutions per minute and the temperature monitored. The mixture will begin to heat up by exothermic reaction and no heat is to be applied to the reactor until the temperature in the reactor ceases to rise. Then the mixture temperature should be increased in 5 degrees C increments per ½ hour until the mixture reaches 60 degrees C. The reaction should be continued until the %NCO = 1.3 % . The target % NCO is reached when every hydroxyl group in the mixture is reacted with an NCO group. Ideally, the result is a single thiol endcapped with two diisocyanates. This outcome can be enhanced by slow addition of the diol to the diisocyanate. The addition should be in 10 g increments, added when the exotherm from the previous addition has ceased. However, chain extended variations of the above ideal outcome are useful, their primary disadvantage being that the product is slightly higher in viscosity. The ideal %NCO is calculated by dividing the weight of the functional isocyanate groups (3 X 42 Dalton) per product molecule by the total weight of the product molecule (9199 Dalton + 3X174 Dalton) yielding approximately 1.3%. Multranol 9199 is available from Bayer (Pittsburgh, PA).

### Example 27: Polyol triisocyanate from polyol diol functional groups for synthesizing ascorbyl palmitate hydrogel.

Any of the diisocyanates prepared above can be trimerized by the addition of a low molecular weight triol such as polycin T-400 or trimethylolpropane (TMP). In this example TMP is used, but the method is adaptable to any triol. Complete trimerization of the diisocyanates will result in viscous products. To yield a lower viscosity product propylene carbonate can be employed or less triol can be used. In the later case, a mixture of diisocyanate and triisocyanate is obtained.

In this example the product of Example 25 is used as the polyether diisocyanate. One equivalent of Example 26 (682 g) is combined with 0.1 equivalent TMP (44.7 g) at room temperature (22 ° C). The mixture is stirred at 100 revolutions per minute and the temperature monitored. The mixture will begin to heat up by exothermic reaction and no heat is to be applied to the reactor until the temperature in the reactor ceases to rise. Then the mixture temperature should be increased in 5 ° C increments per ½ hour until the mixture reaches 60 ° C. The reaction should be continued until the %NCO = 5.8 % . The target % NCO is reached when every hydroxyl group in the mixture is reacted with an NCO group. The ideal %NCO is calculated by dividing the weight fraction of the functional isocyanate groups 10%(3 X 42 Dalton) and 90%(2X42) per product molecule by the total weight fraction of the product molecule (3X1364 Dalton + 134 Dalton) + 1364 yielding approximately 0.3 % + 5.5% = 5.8%. TMP is available from Sigma-Aldrich (Milwaukee, WI).

### EXAMPLE 28: Nitroso hydrogels

Any of the examples 1-27, where ascorbyl palmitate is substituted with 2,3,5,6-tetramethylnitosobenzene.

### EXAMPLE 29: Nitroso hydrogels

Any of the examples 1-27, where ascorbyl palmitate is substituted with 3,5-dibromo-4-nitrosobenzene sulfonate.

### Example 30: Nitrone hydrogels

Any of the examples 1-27, where ascorbyl palmitate is substituted with alpha-(4-pyridyl-1-oxide) n-t-bu nitrone

## Claims

1. An implantable medical hydrogel comprising a polymeric backbone, water and at least one free radical scavenger, wherein the polymeric backbone comprises polyurethane, a copolymerization product of alternating hydrophobic and hydrophilic blocks, and a diisocyanate linker wherein the diisocyanate linker is bound to side chains of the polymeric backbone and said free radical scavenger, wherein the sidechains are comprised of 75% polyethylene oxide and 25% polypropylene oxide units, wherein the free radical scavenger(s) is a spin trap, selected from the group of nitrone compounds and nitroso compounds.

2. The implantable medical hydrogel of claim 1, wherein the free radical scavenger is selected from the group consisting of 2,3,5,6-tetramethyhitrosobenzene, 3,5-dibromo-4-nitrosobenzene sulfonate, alpha-(4-pyridyl-1-oxide) n-t-bu nitrone.

3. The implantable medical hydrogel of claim 1, wherein the polymeric backbone is a crosslinked polyurethane or crosslinked polyurea-urethane.

4. The implantable medical hydrogel of claim 1, wherein the polymeric backbone is bioabsorbable.

5. The implantable medical hydrogel of claim 1, wherein the covalent bond between the free radical scavenger and polymeric backbone is at least one of a urethane link and a urea link.

6. The implantable medical hydrogel of claim 1, wherein the polymeric backbone is terminated with said free radical scavenging group.

7. The implantable hydrogel of any one of claims 1 to 6 for use in a method of treating post-operative tissue adhesions, the method comprising delivery of the hydrogel on an implantable prosthetic.

8. The implantable hydrogel for use in a method of claim 7, wherein the water content of the hydrogel is reduced such that when said hydrogel is used in the method the hydrogel swells by drawing bodily fluids into the volume of the hydrogel mass.

9. The implantable hydrogel for use in a method of claim 7, wherein the implantable prosthetic is a soft tissue reinforcement device.

10. The implantable hydrogel for use in a method of claim 7, wherein the implantable prosthetic is a solid anti-adhesion device.

11. The implantable hydrogel of any one of claims 1 to 6 for use in a method of treating post-operative tissue adhesions wherein said hydrogel reduces the amount of free radicals present in living tissue by chemically bonding free radicals present in living tissue to free radical scavenging groups present in said hydrogel and by mechanically absorbing free radicals present in living tissue into the bulk volume of said hydrogel.

12. The implantable hydrogel for use in a method of claim 11, wherein the hydrogel comprises at least one biofunctional compound(s) dissolved in the water fraction of said hydrogel such that exchange of water between the living tissue and said hydrogel results in release of said biofunctional compound(s) into said living tissue.

## Patentansprüche

1. Implantierbares medizinisches Hydrogel, das ein polymeres Rückgrat, Wasser und wenigstens einen freien Radikalfänger umfasst, wobei das polymere Rückgrat Polyurethan, ein Copolymerisationsprodukt aus alternierenden hydrophoben und hydrophilen Blöcken und einen Diisocyanatverknüpfer umfasst, wobei der Diisocyanatverknüpfer an Seitenketten des polymeren Rückgrats und an den freien Radikalfänger gebunden ist, wobei die Seitenketten aus 75 % Polyethylenoxid- und 25 % Polypropylenoxideinheiten bestehen, wobei der/die freie(n) Radikalfänger eine Spinfalle ist/sind, die aus der Gruppe von Nitronverbindungen und Nitrosoverbindungen ausgewählt ist.

2. Implantierbares medizinisches Hydrogel nach Anspruch 1, wobei der freie Radikalfänger aus der Gruppe ausgewählt ist, die aus 2,3,5,6-Tetramethylnitrosobenzol, 3,5-Dibrom-4-nitrosobenzolsulfonat, Alpha-(4-pyridyl-1-oxid)-n-t-bu-nitron besteht.

3. Implantierbares medizinisches Hydrogel nach Anspruch 1, wobei das polymere Rückgrat ein vernetztes Polyurethan oder vernetztes Polyharnstoff-Urethan ist.

4. Implantierbares medizinisches Hydrogel nach Anspruch 1, wobei das polymere Rückgrat bioabsorbierbar ist.

5. Implantierbares medizinisches Hydrogel nach Anspruch 1, wobei die kovalente Bindung zwischen dem freien Radikalfänger dem polymeren Rückgrat eine Urethanverknüpfung und/oder eine Harnstoffverknüpfung ist.

6. Implantierbares medizinisches Hydrogel nach Anspruch 1, wobei das polymere Rückgrat mit der freien Radikalfängergruppe beendet ist.

7. Implantierbares Hydrogel nach einem der Ansprüche 1 bis 6 zur Verwendung in einem Verfahren zum Behandeln von postoperativen Gewebeadhäsionen, wobei das Verfahren eine Abgabe des Hydrogels auf einer implantierbaren Prothese umfasst.

8. Implantierbares Hydrogel zur Verwendung in einem Verfahren nach Anspruch 7, wobei der Wassergehalt des Hydrogels derart reduziert ist, dass, wenn das Hydrogel in dem Verfahren verwendet wird, das Hydrogel durch Ansaugen von Körperflüssigkeiten in das Volumen der Hydrogelmasse anschwillt.

9. Implantierbares Hydrogel zur Verwendung in einem Verfahren nach Anspruch 7, wobei die implantierbare Prothese eine Weichgewebeverstärkungsvorrichtung ist.

10. Implantierbares Hydrogel zur Verwendung in einem Verfahren nach Anspruch 7, wobei die implantierbare Prothese eine feste Antiadhäsionsvorrichtung ist.

11. Implantierbares Hydrogel nach einem der Ansprüche 1 bis 6 zur Verwendung in einem Verfahren zum Behandeln von postoperativen Gewebeadhäsionen, wobei das Hydrogel die Menge an freien Radikalen, die in lebendem Gewebe vorhanden sind, durch ein chemisches Binden von freien Radikalen, die in dem Hydrogel vorhanden sind, an freie Radikalfängergruppen, die in dem Hydrogel vorhanden sind, und durch mechanisches Absorbieren von freien Radikalen, die in lebendem Gewebe vorhanden sind, in das Gesamtvolumen des Hydrogels verringert.

12. Implantierbares Hydrogel zur Verwendung in einem Verfahren nach Anspruch 11, wobei das Hydrogel wenigstens eine biofunktionelle Verbindung umfasst, die in der Wasserfraktion des Hydrogels derart gelöst ist, dass der Wasseraustausch zwischen dem lebenden Gewebe und dem Hydrogel zu einer Freisetzung der biofunktionellen Verbindung(en) in das lebende Gewebe führt.

## Revendications

1. Hydrogel médical implantable comprenant un squelette polymère, de l'eau et au moins un phagocyte de radical libre, dans lequel le squelette polymère comprend du polyuréthane, un produit de copolymérisation de blocs hydrophobes et hydrophiles alternés, et un lieur diisocyanate, le lieur diisocyanate étant lié aux chaînes latérales du squelette polymère et audit phagocyte de radical libre, les chaînes latérales étant constituées d'unités d'oxyde de polyéthylène à 75 % et d'oxyde de polypropylène à 25 %, le ou les phagocytes de radical libre étant un piège à spin, sélectionné dans le groupe des composés nitrone et des composés nitroso.

2. Hydrogel médical implantable selon la revendication 1, dans lequel le phagocyte de radical libre est sélectionné dans le groupe constitué de 2,3,5,6-tétraméthylnitrosobenzène, de sulfonate 3,5-dibromo-4-nitrosobenzène, de nitrone n-t-bu alpha-(4-pyridyl-1-oxyde).

3. Hydrogel médical implantable selon la revendication 1, dans lequel le squelette polymère est un polyuréthane réticulé ou un polyurée-uréthane réticulé.

4. Hydrogel médical implantable selon la revendication 1, dans lequel le squelette polymère est bioabsorbable.

5. Hydrogel médical implantable selon la revendication 1, dans lequel la liaison covalente entre le phagocyte de radical libre et le squelette polymère est une liaison uréthane et/ou une liaison urée.

6. Hydrogel médical implantable selon la revendication 1, dans lequel le squelette polymère se termine par ledit groupe de phagocytes de radical libre.

7. Hydrogel implantable selon l'une quelconque des revendications 1 à 6, à utiliser dans un procédé de traitement des adhérences tissulaires post-opératoires, le procédé comprenant l'administration de l'hydrogel sur une prothèse implantable.

8. Hydrogel implantable à utiliser dans un procédé selon la revendication 7, dans lequel la teneur en eau de l'hydrogel est réduite de sorte que lorsque ledit hydrogel est utilisé dans le procédé, l'hydrogel gonfle en aspirant les fluides corporels dans le volume de la masse d'hydrogel.

9. Hydrogel implantable à utiliser dans un procédé selon la revendication 7, dans lequel la prothèse implantable est un dispositif de renforcement des tissus mous.

10. Hydrogel implantable à utiliser dans un procédé selon la revendication 7, dans lequel la prothèse implantable est un dispositif anti-adhérence solide.

11. Hydrogel implantable selon l'une quelconque des revendications 1 à 6, à utiliser dans un procédé de traitement des adhérences tissulaires post-opératoires, dans lequel ledit hydrogel réduit la quantité de radicaux libres présents dans le tissu vivant en liant chimiquement les radicaux libres présents dans le tissu vivant à des groupes de phagocytes de radical libre présents dans ledit hydrogel et en absorbant mécaniquement les radicaux libres présents dans le tissu vivant dans le volume apparent dudit hydrogel.

12. Hydrogel implantable à utiliser dans un procédé selon la revendication 11, dans lequel l'hydrogel comprend au moins un ou plusieurs composés biofonctionnels dissous dans la fraction aqueuse dudit hydrogel de sorte que l'échange d'eau entre le tissu vivant et ledit hydrogel entraîne la libération dudit ou desdits composés biofonctionnels dans ledit tissu vivant.
